# EUROPEAN PATENT SPECIFICATION

(11) **EP 1 799 626 B1**
(45) Date of publication and mention of the grant of the patent: **18.01.2012**
(21) Application number: 05795077.6
(22) Date of filing: 10.10.2005
(51) Int. Cl.: C04B 40/04

(54) **MULTIPLEXED PROTEIN ADSORPTION ASSAY**
GEMULTIPLEXTER PROTEINADSORPTIONS-ASSAY
ANALYSE MULTIPLEX DE L'ADSORPTION DE PROTEINES

(30) Priority: 12.10.2004 US 617192 P
(43) Date of publication of application: 27.06.2007
(73) Proprietor: Schott AG, 55122 Mainz (DE)
(72) Inventor: BURZIO, Luis, Mountain Top, PA 18707 (US); CONZONE, Samuel, David, Clarks Summit, PA 184411 (US); HAINES, Daniel, Edward, Lake Ariel, PA 18436 (US); HORMES, Robert, 79279 Müllheim (DE); KOLLER, Horst, CH-9032 Engelburg (CH); PFEIFER, Joachim, 95448 Bayreuth (DE)
(74) Representative: Schnabel, Jörg
(86) International application number: PCT/EP2005/010882
(87) International publication number: WO 2006/040107

(56) References cited:
- WO-A-99/28504
- US-A1- 2003 134 033
- US-A1- 2005 145 048
- BRYNDA EDUARD ET AL: "Albumin and heparin multilayer coatings for blood-contacting medical devices" JOURNAL OF BIOMEDICAL MATERIALS RESEARCH, vol. 51, no. 2, August 2000 (2000-08), pages 249-257, XP002370547 ISSN: 0021-9304
- HORMES R: "Coating of borosilicate glass for the improvement of the chemical stability of glass containers" PHARMAZEUTISCHE INDUSTRIE 2003 GERMANY, vol. 65, no. 9 A, 2003, pages 951-955, XP009062500 ISSN: 0031-711X

## Description

Proteins have a strong affinity for the surfaces with which they come in contact. In pharmaceutical packages and medical devices this affinity often results in the loss of valuable proteins due to surface adsorption. Surface adsorption is governed by many factors including the nature of a protein, the character of the surface and the additives in the protein solution. Pharmaceutical packagers frequently overfill a container to account for protein loss due to absorption. Many attempts have been made to provide surfaces that resist protein adsorption. No one product can meet the needs of all protein solutions and package performance is highly unpredictable partly because the amount of protein absorption is dependent upon so many factors such as, for example, the pH of the solution, the surface coating and the nature and concentration of the protein. In the past, each new pharmaceutical solution was tested against specific packages or devices and the amount of protein left in the solution was measured to determine the protein loss due to absorption.

Proteins are a heterogeneous class of biomolecules with widely varying physicochemical characteristics. Some general observations regarding the strength of interaction between proteins and surfaces can be made.

**Table I: Ideal characteristics of surfaces resistant to protein adsorption/denaturation**

| Desirable Surface Characteristic | Rationale |
|---|---|
| Non-Ionic | A non-ionic surface is electrostatically neutral and will not attract or ionically bind to proteins, which contain both positively and negatively charged motifs. |
| Sterically hindering | A surface containing, e.g., flexible polymeric dendrites provides a flexible, protective barrier at the glass suface and precludes intimate glass/protein contact, thus preventing adhesion and denaturation. |
| Hydrophilic | A hydrophilic surface promotes the formation of a compact H₂O-rich layer (Stern layer) at the surface of the substrates, e.g., glass which prevents the more hydroscopic proteins in solution from coming into direct contact with the, e.g., glass substrate. |
| Hydrogen bond accepting | A hydrogen bond accepting surface will form hydrogen bonds with H₂O molecules found within the Stern layer, which will further prevent proteins from interacting directly with the substrate. |

A problem with current pharmaceutical packaging products or medical devices is that no one product possesses each of the positive traits that are needed to provide comprehensive, protein-deterring characteristics. This "mixed bag" of desirable/undesirable characteristics (Table II) renders the performance of products highly unpredictable for universal, protein-based pharmaceutical packaging applications. This unpredictability is evident when considering the highly contradictory results from various "protein adsorption" or "protein loss" studies, which date back to at least 1998. This unpredictability and lack of knowledge about how to truly prevent protein adsorption/loss is ultimately manifested in the inability of the pharmaceutical packaging or medical device industries to develop a single low-loss, protein inhibiting packaging product or device for the pharmaceutical or medical device industry.

**Table II: Assessing surface characteristics**

| Desirable Characteristic | Type I/Type I-Plus Glass | Siliconized Glass/Plastic | TopPac Plastic |
|---|---|---|---|
| Non-Ionic | High negative surface charge at neutral pH that promotes interaction with proteins | relatively non-ionic | relatively non-ionic |
| Sterically hindering surface characteristics | No steric hindrance characteristics | Steric hindrance possible from silicone chains | No steric hindrance characteristics |
| Hydrophilic | Cleaned glass surfaces are hydrophilic | Silicone oils are generally highly hydrophobic | TopPac is an aliphatic, aromatic co-block polymer and thus hydrophobic |
| Hydrogen bond accepting | A glass surface will accept hydrogen bonding | Poor hydrogen bonding characteristics | Poor hydrogen bonding characteristics |

Type I is a product line of Schott-forma vitrum made from borosilicate glass with the highest class of hydrolytic resistance; Type I Plus is product line of Schott-forma vitrum with glass receptacles with a silicon oxide coating; TopPac is a product line of Schott-forma vitrum with polymer receptacles made of cyclic olefin copolymer like Topas (marked by Ticona)

The contradictory and sometimes confusing results from previous "protein loss" and "protein adsorption" studies have been further compounded by differences in the testing procedures and assays utilized for assaying "loss" and/or "adsorption", In previous testing, variability in the testing parameters, including duration of the study, concentration of the protein, testing temperature, pH, use of detergents/additives, etc. have rendered final interpretation and comparison of results difficult or impossible. Further, most "protein loss" assays were conducted using techniques, such as the Bicinchoninic acid (BCA) technique which only allows for the determination of the protein concentration after an adsorption process, but provides no insight into where the proteins were preferentially adsorbed within the pharmaceutical package.

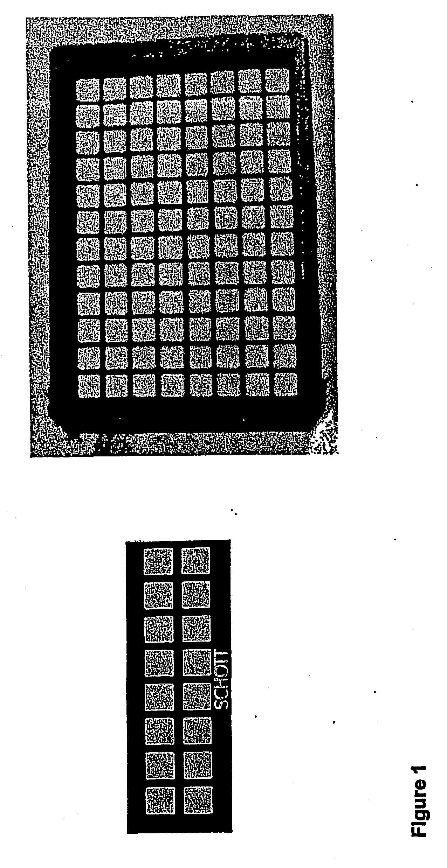

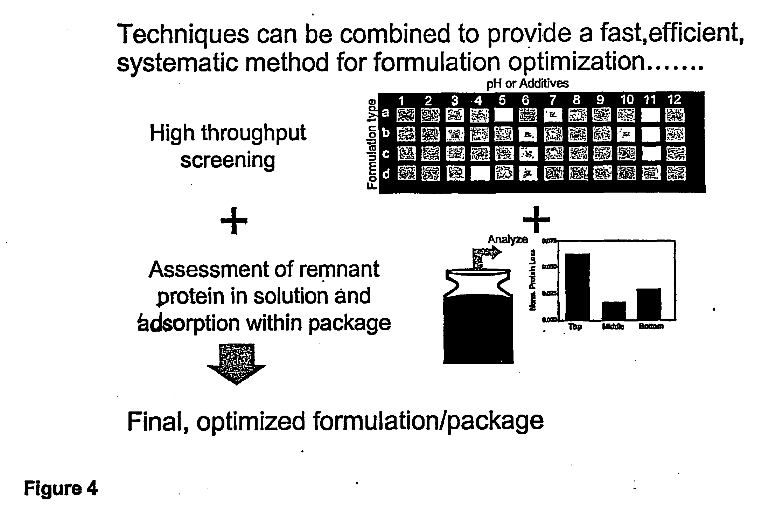

The assay of the present invention will enable drug formulators, medical device developers and pharmaceutical packaging developers to optimize formulations and material surfaces to inhibit the irreversible adsorption of drug compound while utilizing small quantities of compound containing solutions (<<1ml) and small amounts of potential pharmaceutical packaging or device materials (surface areas <1mm²). Further, such testing can be achieved in a multiplexed manner (i.e., 2 to 10,000's of formulation/well surface combinations can be assessed on a single, chip-based platform) as shown in Figure 1. Further, such testing can be combined with more thorough testing in actual full scale pharmaceutical packages to fully characterize the performance and stability of a drug compound, e.g., with respect to an identified surface candidate, as shown in Figure 4, thus providing a total solution for pharmaceutical packaging or medical device optimization.

The assay enables a pharmaceutical packager to simultaneously directly compare the adsorption behavior of a specific protein (e.g., recombinant drug, cytokine, enzyme etc.) in a solution containing various specific additives (e.g., buffers etc.) under various specific conditions (e.g., pH, temperature etc.) against a variety of potential substrate surface coatings (e.g., silica, polymer coated glass etc.). The multiplexed assay enables the packagers to simultaneously target the specific packaging conditions (e.g., surface coating, pH, additives) that will result in the least amount of protein adsorption and thus product loss.

Generally, the present invention relates to a multiplexed assay that allows simultaneous measurement of the adsorption interaction of one or more protein solutions with one or more substrate surfaces, as defined in claim 1. Briefly, a substrate is divided into multiple wells, each of which has a surface to be tested, e.g., the substrate surface per se or one which is coated or treated in some fashion. Each well of the multiple well substrates is then subjected to, i.e., exposed to a protein solution and the level of protein adsorption in each of said wells is determined. (The term "adsorption" is not intended to place any limitation on the nature of the interaction between the assayed component of a solution and the test surface. As long as the interaction is sufficient to keep the component in association with the surface sufficiently to be detected in an assay, it is included within the scope of the term.)

The substrate is a multiwell substrate. Each substrate may contain from 2 to greater than 10,000 wells that are created, e.g., with a hydrophobic patterning material. Preferably, the substrate contains greater than 4 wells per substrate. More preferably, the substrate contains greater then 8 wells per substrate. Most preferably, the substrate contains greater then 16 wells per substrate. The protein solutions may contain buffers, salts, stabilizers, preservatives, acids and/or bases, etc., as are common in the pharmaceutical industry. Typically, the protein to be tested is an antibody, an enzyme, recombinant erythropoietin, a recombinant hormone, polypeptides in general, peptides, vaccines, etc. The level of protein adsorption in each of said wells may be determined by, for example, incubating the wells with labeled antibodies and scanning to determine the amount of protein bound. Alternatively, the level of protein adsorption in each of said wells may be determined by, for example, interrogation with an enzyme conjugated antibody and measuring the signal amplification. Thus, the amount of protein that adsorbs to the substrate surfaces under various conditions and various solution parameters can be easily determined.

### BRIEF DESCRIPTIONS OF THE DRAWINGS

Various features and attendant advantages of the present invention will be more fully appreciated as the same becomes better understood when considered in conjunction with the accompanying drawings, in which like reference characters designate the same or similar parts throughout the several views, and wherein:
Figure 1 shows an image of a partitioned microscope slide format (left) and microtiter plate format (right). Such formats can be designed to contain from about 2 to >1000 individual wells.
Figure 2 shows a pictorial representation that demonstrates how treated well surfaces can interact with drug compounds immersed in solution.

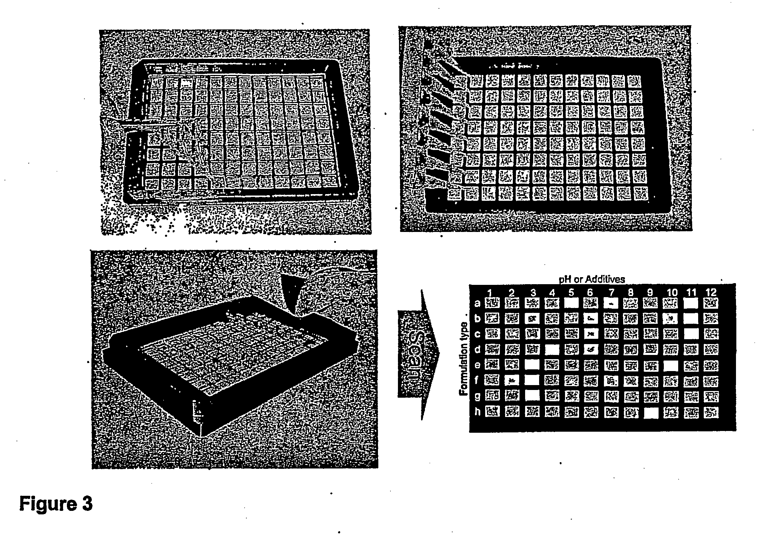

Figure 3 depicts the steps involved in a typical assay. First (top left) a silicone-based superstructure is applied to the patterned substrate. Second (top right); solutions containing the protein drug compound(s) are added. Third (bottom left), a sealing strip is applied to the top of the device to inhibit evaporation. Finally (bottom, right), after washing and labeling, the amount of adsorbed protein is quantified via fluorescent scanning.Figure 4 shows a pictorial representation of one possible total pharmaceutical packaging development process, whereby multiplexed assays are first used to screen for optimal packaging conditions, and later followed by packaging-specific tests to achieve a final formulation.
Figure 5 is a pictorial representation showing how different techniques could be used in conjunction to quickly and efficiently provide packaging/formulation solutions to the pharmaceutical industry.
Figure 6 depicts a variety of contemplated patterning designs for use in the assaying applications of the present invention.
Figure 7 depicts direct and indirect assay methods developed to determine protein adsorption to surfaces.
Figure 8 shows the effect of different pHs on the ionic attraction of IgG to glass surfaces.
Figure 9 shows the adsorption of different proteins to glass. Five proteins are used: IgG (G), Insulin (I), Histone (H), Fibrinogen (F), C. Anhydrase (C). They are incubated at 10µg/ml at 3 different pHs (5, 7 and 9).
Figure 10 shows the effect of positively charged surfaces on the binding of positive and negative charged proteins.
Figure 11 depicts the optimization of protein formulations on incubated glass slide wells with a protein in different buffers (A) and with and without Tween 20 (B).

Surfaces susceptible to protein adsorption include pharmaceutical packaging components (e.g., glass vials, ampoules, stoppers, caps, ready to fill syringes--glass and plastic, cartridge-based syringes, pure silica-surfaced vials, plastic-coated glass vials, plastic and glass storage bottles, pouches, pumps, sprayers and pharmaceutical containers of all types) and medical devices (e.g., catheters, stents, implants, syringes etc). Any candidate surface which is considered for contact with a protein and is susceptible to protein adsorption can be assayed.

Typically, the assay substrate material is glass or plastic. Preferably the substrate material is a SiO₂ based glass slide. Most preferably the glass comprises a commercially relevant glass which is used in pharmaceutical packaging applications such as, for example, one comprising 65-85 wt % SiO₂, 3-20 wt % B₂O₃, 0-20 wt % Al₂O₃, 1-15 wt % Na₂O, 1-15 wt % K₂O, 0-10 wt % MgO, 0-10 wt % CaO and 0-10 wt % BaO.

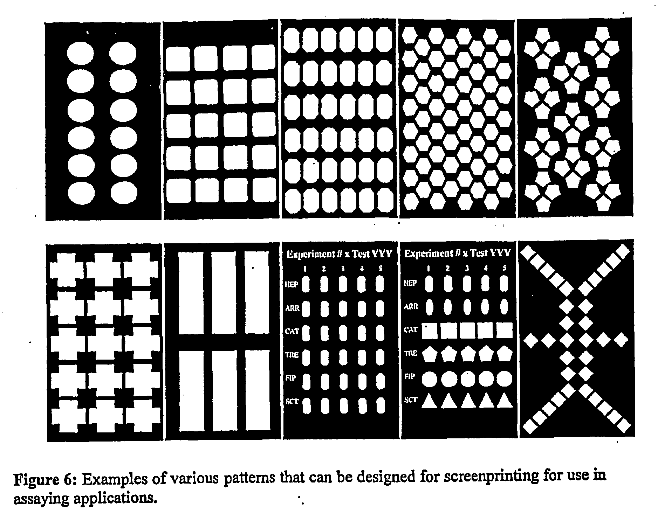

A variety of known commercially available patterning compositions such as PTFE polymer (Poly Tetra Flourine Ethylene), fluoropolymers or silicone can be applied to the substrate to create hydrophobic boundary regions thereby creating wells. The boundary regions (i.e., walls) provide a barrier between each well surface (bottom, typically) where assaying reactions can be conducted. Figure 6 depicts numerous well patterns that would be useful in the assay and methods of the present invention. The pattern design itself is flexible, being limited only to the human imagination and the limitations of graphics programs used to make symmetrical or unsymmetrical geometric patterns (repeating or non-repeating over the substrate surface) that include ovals, squares, rectangles, stars, etc. that can be adapted to the experimental assaying design as needed. The wells may or may not be interconnected to provide a means for interaction between two or more wells on a substrate. The patterning of the substrate into wells allows processing of multiple assays in parallel rather than serially. Patterned substrates, such as those disclosed in US 10/778,332 titled "Low-Fluorescent, Chemically Durable Hydrophobic Patterned Substrates for the Attachment of Biomolecules," ensure physical separation between the various relevant protein solutions being tested without the worry of cross contamination between assays. The patterning material is deposited by methods known in the art. Preferably the patterning material is screen-printed onto a glass substrate to provide distinct boundary and well regions.

It is contemplated that wells of the patterned substrate can in one embodiment remain uncoated and untreated, with just the underlying substrate composition exposed to the various protein solutions. When the well area has not been additionally coated or treated, then the substrate surface for that well will be an untreated substrate surface. Alternatively, each well can be treated or coated with a different test coating or surface treatment. Or some of the wells may be treated and others may be coated. Obviously a large variety of combinations of well coatings and/or treatments can be tested on a single substrate.

Substrate surfaces comprise uncoated, coated, treated or untreated well surface areas such as, for example:
1) Glass (e.g., silicates, borates, borosilicates, phosphates, etc);
2) Glass that has been heat-treated with an oxy-fuel flame to emulate the processing utilized to convert glass tubing into pharmaceutical packaging;
3) Polymeric materials, such as acrylics, polycarbonates, polyesters, polypropylenes, polyacetals, polystyrenes, polyamides, polyacrylamides, polyimides, polyolefins, cyclic olefin copolymers, especially bicyclic olefin copolymers, or polymeric films;
4) Organic coatings having the following functional group(s) present at the well surface: amine, epoxide, isocyanate, isothiocyanate, acyl azide, N-hydroxysuccinimide (NHS) ester, sulfo-NHS ester, sulfonyl chloride, imidoester, carbodiimide, acid anhydride, iodoacetyl, malemide, aziridine, acryloyl, disulfide, diazoacetate, aryl azide, thiol (sulfhydryl), mercapto, acetate, hydroxyl, carbonate, aldehyde, alkane, alkene, carboxylate, esters, ethers, etc. - (this is a non-exhaustive list of potential functional groups); organic coatings/films composed of dendrimers, polymers (polyethylene glycols - PEG), nanoparticles, and hyper-branched polymers, e.g., that contain the aforementioned functional groups;
5) Metallic coatings such as gold, silver, platinum, palladium, etc;
   and/or
6) Inorganic oxide coatings such as SiO₂, TiO₂, ZrO₂, Al₂O₃, etc.

Many other possibilities exist, e.g., other surfaces used in the pharmaceutical packaging and medical device fields.

Other typical surface treatments or coatings include polymer coated surfaces, native glass, etched glass, thermal treated glass, borosilicate glass coated with a PEG layer, Hyal, siliconized glass or plastic, TopPac, Type 1, Type 1 plus. It is contemplated that any pharmaceutical packaging or medical device surface, surface treatment or coating can be tested against the various parameters of protein solutions. There is a wealth of general knowledge regarding surfaces and or coatings that resist protein adsorption. See, for example, Emanuele Ostuni, Lin Yan, George M. Whitesides- Colloids and Surfaces Biointerfaces 1999, 15, 3-30. Additionally, there is a wealth of general knowledge regarding surfaces that are designed to decrease protein adsorption. See, for example, Emanuele Ostuni, Robert G. Chapman, R. Erik Holmin, Shuichi Takayama, George M. Whitesides- Langmuir 2001, 17, 5605-5620. A large variety of surface coating combinations can exist on a single substrate. All of these represent the large number of available surface treatment and/or coating possibilities.

As used herein, the term "protein solution" refers to a particular protein of interest in the presence of (typically) an aqueous solution that may contain various additives. Typical protein solutions to be tested include pharmaceutically relevant moieties such as cells, tissues, and derivatives thereof. Among the proteins are included any polyaminoacid chain, peptides, protein fragments and different types of proteins (e.g., structural, membrane, enzymes, antigens, monoclonal antibodies; polyclonal antibodies, ligands, receptors) produced naturally or recombinantly, as well as the derivatives of these compounds, etc. Specific protein drugs include antibodies (e.g. Remicade and ReoPro from Centocor; Herceptin from Genentech; Mylotarg from Wyeth, Synagis from MedImmune), enzymes (e.g. Pulmozyme from Genentech; Cerezyme from Genzyme), recombinant hormones (e.g., Protropin from Genentech, Novolin from Zymogenetics, Humulin from Lilly), recombinant interferon (e.g., Actimmune from InterMune Pharmaceutical; Avonex from Biogenldec, Betaseron from Chiron; Infergen from Amgen; Intron A from Schering-Plough; Roferon from Hoffman-La Roche), recombinant blood clotting cascade factors ( e.g.,TNKase from Genentech; Retavase from Centocor; Refacto from Genetics Institute; Kogenate from Bayer) and recombinant erythropoietin (e.g., Epogen from Amgen; Procrit from J&J), and vaccines (e.g., Engerix-B from GSK; Recombivax HB from Merck & Co.).

Typically drug compounds to be tested will be immersed within an aqueous solution that may contain various additives. Such additives have an influence on the binding of a protein drug to a packaging or device surface. Typical additives include buffers (e.g., phosphate, Tween, citrate, and/or acetate), salts such as sodium chloride at physiological concentrations, stabilizers (e.g., anti-oxidants such as histadine, chelators such as EDTA, human albumin or glycerin etc.), preservatives (e.g., phenol, metacresol, benzyl alcohol etc.), and acid or bases (e.g., citric acid, sodium hydroxide, hydrochloric acid, acetic acid etc) to adjust the pH of the formulation to physiologically safe levels.

The patterned and treated substrate can be used to simultaneously investigate the interaction between multiple relevant protein solution parameters (duration, temperature, concentration, pH, etc.) and a variety of surface coatings/treatments using very small amounts of protein. After the assay is completed, the amount of protein adsorbed on each well of the patterned substrate can be detected using known commercially available detection methods for protein adsorption.

The most common analytical techniques for determining protein adsorption take advantage of the change in optical and/or electrical properties of a surface that has adsorbed proteins. These techniques provide a measurement of the presence/absence of species on a surface. Some techniques allow determination of additional information as to the amount or thickness of adsorbed protein (SPR; ellipsometry; QCM; XPS; radioactive isotopic labeling; solute depletion; fluorescence emission spectroscopy), conformation (ATR FT-IR; Raman scattering; XPS; low angle X-ray reflectivity; scanning force microscopy), or binding energy to the surface (scanning force microscopy). Surface plasmon resonance (SPR) is very sensitive to changes in the index of refraction at and near the surfaces of metal films. SPR can measure the before/during/after protein adsorption to determine kinetic and thermodynamic information regarding the adsorption of proteins. See, for example, Jennifer M. Brockman, Anthony G. Frutos, Robert M. Corn- J. Am. Chem. Soc. 1999, 121, 8044-8051. Ellipsometry can be used to determine if proteins have adsorbed to a surface by measuring the change in the index of refraction before/after protein adsorption to give an experimental thickness of the layer of proteins adsorbed. This detection method is useful if a substrate has a refractive index different from the coating. See, for example, Delana A. Nivens, David W. Conrad -Langmuir 2002, 18, 499-504; M. Mrksich, L. E. Dike, J. Tien, D. E. Ingber, G. M. Whitesides- Exp. Cell Res. 1997, 235, 305-313; and Kevin L. Prime, George M. Whitesides -J. Am. Chem. Soc. 1993, 115, 10714-10721. Quartz crystal microbalance (QCM) measures changes in the fundamental frequency of vibration for a quartz crystal for protein adsorption via the piezoelectric effect, yielding adsorbed protein layer thickness. Surface acoustic wave (SAW) and acoustic plate mode (APM) devices takes advantage of changes in surface acoustic waves (velocity and amplitude) when proteins adsorb to the surface of a crystal modified with electrodes, detecting the presence or absence of protein binding. See, for example, Robert Ros Seigel, Philipp Harder, Reiner Dahint, Michael Grunze, Fabien Josse - Anal. Chem. 1997, 69, 3321-3328). X-ray photoelectron spectroscopy (XPS) uses X-rays to eject electrons from atoms; each atom has different XPS spectrum and allows determination of the number and type of atoms per unit area. XPS can also be used to determine if protein has adsorbed to a surface by measuring the spectrum from a protein adsorbed to a surface vs a non-protein adsorbed surface. Attenuated total internal reflectance fourier transfer infrared (ATR FT-IR) spectroscopy examines the twisting, bending, rotating, and vibrational motions of molecules. The spectra provide information that can be used to determine the presence or absence of a protein and give information regarding its conformation on the surface. Low-angle X-ray reflectometry may be used to determine the variations in electron density at an interface and allows resolution of packing differences in layers. Radioactive isotope labeling can be used to quantify the amount of protein adsorbed by ionization detection (Geiger counter) or liquid scintillation. See, for example, Y. S. Lin, V. Hlady and J. Janatova -Biomaterials, 13, (1992), p. 497. Solute depletion measures the amount of protein in solution before or after exposure to a surface. Scanning force microscopy uses a probe tip with a known position to characterize a surface species. The probe tip may be coated with specific molecules to determine chemical and physical interactions with a surface. See, for example, J. N. Lin, B. Drake, A. S. Lea, P. K. Hansma, and J. D. Andrade- Langmuir, 6, (1990), p. 509. Fluorescence emission spectroscopy measures the inherent fluorescence of a molecule or the fluorescence of a fluorescent label on a molecule. Proteins may be fluorescently labeled and detected using fluorimeters. See, for example, V. Hlady, Applied Spectroscopy 1991, 45, 246 and D. J. Sbrich and R. E. Imhof in Topics in Fluorescence Spectroscopy, J. R. Lakowicz Ed., Plenum, New York, (1991), p. 1. Circular dichroism measures the magnitude of polarized light rotation and detects the presence or absence of proteins. See, for example, C. R. McMillin and A. G. Walton- J. Colloid Interface Sci., 84, (1974), p. 345. Raman scattering is complimentary to infrared and measures the vibrational spectrum of molecules that undergo change in polarizability. It is used to determine the presence or absence of specific molecules/functional groups. See, for example, T. M. Cotton in Surface and Interfacial Aspects of Biomedical Polymers, 2, J. D. Andrade Ed., Plenum Press, New York, (1985), p. 161. In general, this invention is not limited in any way by the nature of the forces holding the protein molecules to the substrates.

Fluorescent detection can be utilized as a direct indication as to the amount of a protein bound to a surface. In this method the protein to be studied is conjugated to a fluorescent dye, such as those typically used in DNA and protein microarrays (e.g., Cy-dyes from GE Healthcare, Alexa-flour dyes from Molecular Probes, or other dyes available commercially and used typically to label proteins (dansylamide, flouresceine)). These dyes are normally conjugated to the protein through amine-reactive groups (typically, NHS-esters, aldehydes or epoxides) and can be easily detected. The quantitative amount of labeled antibody can be measured after washing via laser scanning, utilizing any one of the various commercial scanners from Axon, Perkin Elmer, Alpha Innotech, Tecan, Agilent, Affymetrix, etc. utilized for microarray analysis (as shown pictorially in Figure 3). This method is easy to apply to many different proteins, with the major caveat being that the protein is modified, which in some instances may lead to interactions with the surface that may not occur in the unmodified protein. It will also change the charge distribution on the protein since the reactions mostly involve the epsilon-amino group of lysine as well as the amino terminal. An alternative method involves detecting the adsorbed proteins with a labeled antibody. This indirect method involves obtaining specific antibodies to a protein and labeling them with the same fluorophores described above.

If desired, the multiplexed assay of the present invention can be used in conjunction with other protein loss/adsorption assays such as those described in Table III. Once the multiplexed assay has identified desirable formulation and surface combinations, the following assays can be used to test protein loss within a full scale pharmaceutical package or medical device.

**Table III: Techniques identified for assessing protein loss/adsorption**

| TECHNIQUE | DESCRIPTION | PROS | CONS | |
|---|---|---|---|---|
| Multiplexed assay | Fluorescent-labeled proteins are deposited into the wells of a flat substrate, incubated and protein adsorption is measured by fluorescent scanning. | High throughput, low cost, extremely low protein amounts required, direct assay. | Useful for primary screening, as testing is not conducted within a true pharma package. | Utilize this test for rapid investigation of multiple protein-adsorption mechanisms. |
| BCA test | Staining technique based on Cu²⁺ reduction to Cu⁺ in the presence of proteins. Test is performed on protein-containing solutions after being tested within pharmaceutical packaging. | Simple, fast, accurate technique, which is highly accepted in the industry. | Does not allow one to determine where the proteins were "lost" within the pharma package. | Utilize in conjunction with the Amino Acid Assay described below when conducting tests within actual full scale pharmaceutical packages. |
| Amino Acid Assay | Assay for remnant protein adsorbed within the package by hydrolyzing proteins and measuring for amino acid concentration using column chromatography. | Accurate and accepted assay can be used to detect what amount of protein was lost within a package and determine where the majority of protein adhesion occurred. | Not amenable to high throughput screening. | Utilize this technique in conjunction with the BCA test when conducting testing within actual full scale pharmaceutical packages. |

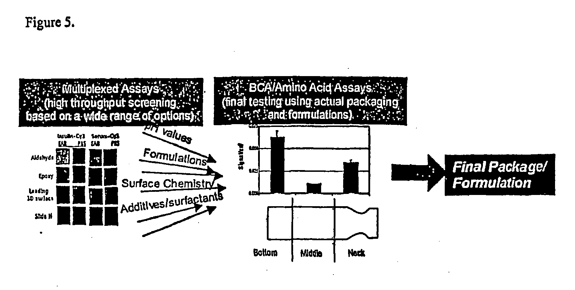

The techniques described above can be used in the approach pictorially described in Figure 5 to accelerate testing and to provide packaging support and therefore solutions to the pharmaceutical and medical device industries.

Thus, the assay of the present invention is useful in allowing packaging and medical device scientists to study the stability of novel new drug compounds such as, for example, small molecules, antibodies, proteins (natural or recombinant), cytokines, vaccines, under a multitude of different packaging and formulation conditions, while consuming very limited amounts of a precious drug compound. Tens of thousands of formulation/well surface combinations can be assessed on a single, chip-based platform. Thus, one can rapidly identify the optimal combination of material surface and product formulation for a given protein-based pharmaceutical compound. The ability to tailor the surface properties of materials and optimize formulations will reduce or eliminate loss of valuable protein due to surface adsorption and allow easy scale-up from assay into a tangible, scalable prototype or a commercial batch.

Although this application is written primarily in terms of proteins, polypeptides or peptides, it can also be applied to other biomolecules such as nucleic acids, polynucleotides (e.g., DNA, RNA, mRNA, pDNA, etc., oligonucleotides), protein/nucleic acid complexes, etc. by straightforward extension application of the invention to biomolecules is routine. Application of this invention to biomolecules is routine. Assay methods and techniques (reagents, signaling methodology, detection methodology, etc.) are all well known.

By "biological specificity" is meant the normal type of biological lock and key type of bonding which is sufficiently unique to identify a species from all others, e.g., antibody-antigen (protein) interactions, receptor-ligand interactions, highly stringent hybridization, etc. Instead, the surface differences here are designed not to identify proteins but to vary adsorption of a protein entity to a treated surface.

Without further elaboration, it is believed that one skilled in the art can, using the preceding description, utilize the present invention to its fullest extent. The preceding preferred specific embodiments are, therefore, to be construed as merely illustrative, and not limitative of the remainder of the disclosure in any way whatsoever.

The entire disclosure[s] of all applications cited herein are incorporated by reference herein.

### EXAMPLE I

### Multiplexed Formulation Optimization Protocol

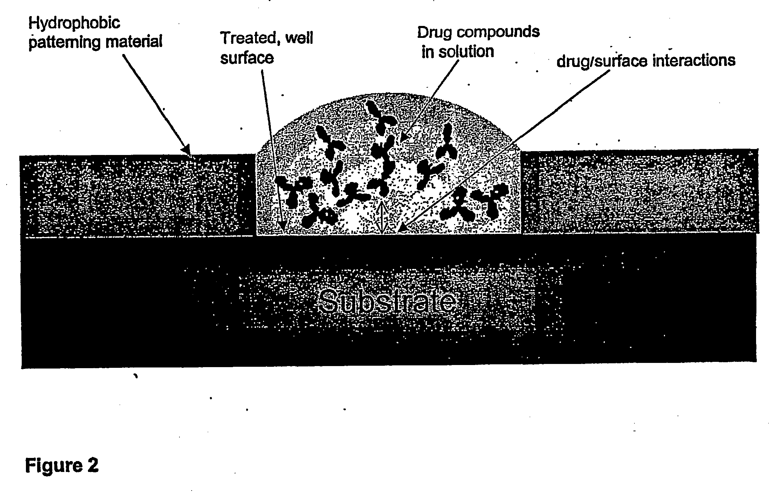

a) Choose a substrate which is ideally in the general shape of a microscope slide (nominally 25x75x1 mm³) or a microtiter plate (substrate within the MTP frame has nominal dimensions of 74x110x1mm³).
b) Partition off the substrate into individual wells (of any shape) with cross sections that can range from <100 µm to several mm. These wells may be formed by, for example, screen-printing a hydrophobic pattern onto the starting substrate, as described in patent application US10/778,332 titled "Low Fluorescent, Chemically Durable Patterned Substrates for the Attachment of Biomolecules." The resulting patterned substrate can take on the general appearance shown by the examples in Figure 1.
c) Interrogate each individual well (Figure 1) with various drug compounds as shown in Figure 2. The drug-compound-containing solution may be placed into each well via milliliter, microliter or nanoliter pipeting.
d) Allow solutions to interact with the well surfaces from a time ranging from 1 sec to 12 months or longer, based on the intent of the study; (e,g., some aging studies may require interaction times of >1year). During aging, a sealed superstructure can be utilized to inhibit evaporation, as shown in Figure 3. If desired, the polymeric-based superstructure can be applied before the aqueous solution is deposited into the wells, followed by the application of a sealing strip that eliminates the risk of evaporation.
e) Characterize or measure the amount of drug compound that has irreversibly adsorbed to the well surfaces. This can be done by various methods described above, such as, for example:
   i) After the protein has been allowed to adsorb, the wells may be incubated with labeled antibodies, washed, and then scanned to determine the amount of protein bound;
      or
   ii) After the protein has been allowed to adsorb, the wells may be interrogated with enzyme conjugate antibodies, so as to allow for signal amplification similar to an ELISA assay;
      or
   iii) Different types of probes can be used to detect the adsorbed proteins including antibodies (mono- and polyclonal), affibodies, antibody fragments, oligonucleotides, amine reactive fluorophores and dyes (e.g. Cy-dyes and others as described above), specific ligands (e.g. detecting biotinylated proteins with fluor-labeled streptavidin), and small molecules that specifically bind to the protein of interest.

### EXAMPLE II

Two types of assays demonstrate the adsorption of proteins in solution to glass surfaces. The use of glass slides divided into wells with a silicone superstructure allows incubation of 100µL volumes of protein solution. To detect and quantify the protein bound to the surface fluorescent dyes are used to ensure adequate sensitivity. Two types of assays, a direct and an indirect assay are discussed.

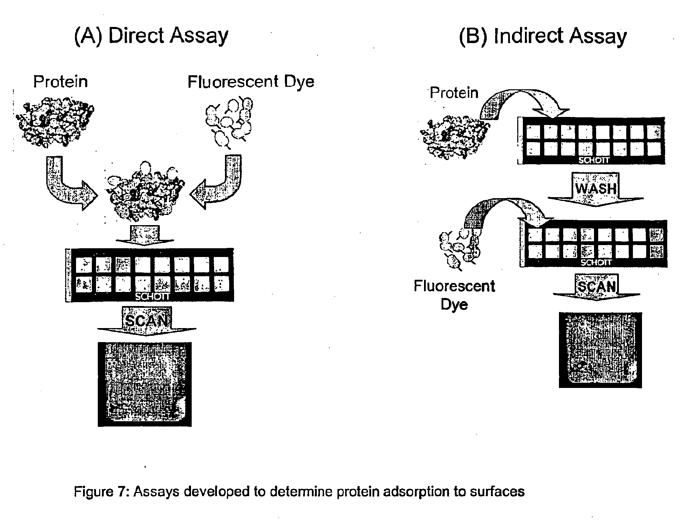

The direct assay is based on protein solutions, where the protein is modified to contain Cyanine dye (Cy3) (see figure 7A). The protein solution is incubated in the slides wells and then removed. The excess protein:dye conjugate is washed with water for injection (WFI); the slide is dried and then scanned using a 532nm laser scanner.
The amount of fluorescent signal is measured throughout the well and the amount of protein is calculated using calibration curves.

In the indirect assay (see figure 7B), unlabelled protein is incubated in the wells. After the incubation period the protein solution is removed and the wells are washed with WFI. A fluorescent dye that contains an NHS-ester is incubated in the well. The dye NHS-ester reacts with the amine groups on the protein and the fluorescent moiety becomes attached to the proteins adsorbed to the surface. The excess unreacted dye is then washed from the well, the well is dried and the slide is scanned as above. The quantification of the protein bound is also done by calibration curves for each specific protein.

### EXAMPLE III

A multiplexed assay is used to assess the adsorption of a protein formulated at different pHs. Proteins have an isoelectric point (pl), which is the pH at which the net charge of the protein is zero. At any pH below the pl the protein will be positively charged, while at pH above the pl the net charge will be negative. Meanwhile the zeta potential for glass is negative at any pH above 3, therefore the glass will be negatively charged above that pH.

Human IgG labeled with Cy3 fluorescent dye (Excitation 532nm, emission: 535nm) in a 100mM Phosphate buffer at pH 5,6,7,8, and 9 is formulated. The pl of IgG is 7.8, therefore at most pH's the protein would be positively charged. 100 µL of the protein solution is incubated in wells formed on slides as described for a period of 72hours. After incubation the slide wells are washed with 100 µL of water for injection (WFI) three times. The slides are then scanned in a laser scanner.

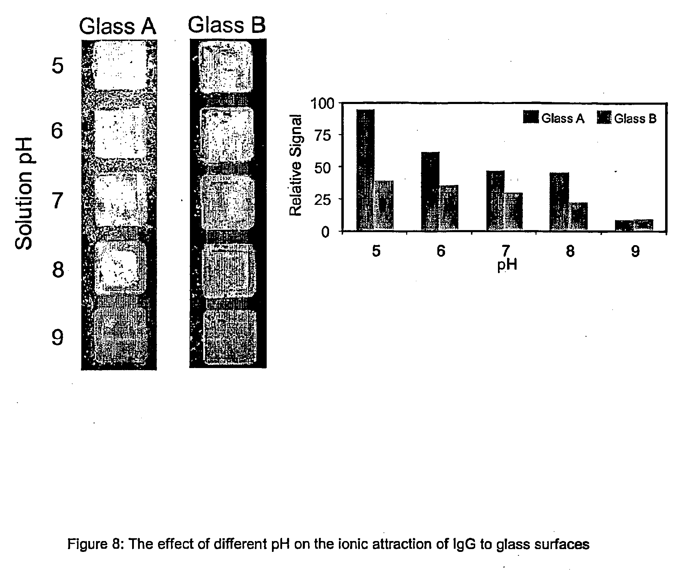

The images shown in figure 8 show the adsorption of the IgG solutions on two types of glass. It can be observed that as the pH increases the amount of IgG-Cy3 adsorbed to the surface decreases due to the increase in negative charge that repels the protein from the negatively charged glass surface. The optimal formulation in this case should be done at pH of around 9 to minimize de binding due to ionic interactions.

### EXAMPLE IV

Given the different nature of proteins in general it is to be expected that different proteins will adsorb to a varying degree to the same surface. In this example the adsorption of different proteins all formulated in the same solutions is tested.

Different aspects of protein characteristics in the proteins selected including large (Fibrinogen, molecular weight 340,000) to small (insulin, molecular weight 5600), acidic pl (albumin, pl 5.2) to basic pl (histone, pl 11.5) are covered. All are formulated in a 100mM phosphate buffer at pH 5, 7, and 9, and incubated as described in the previous example.

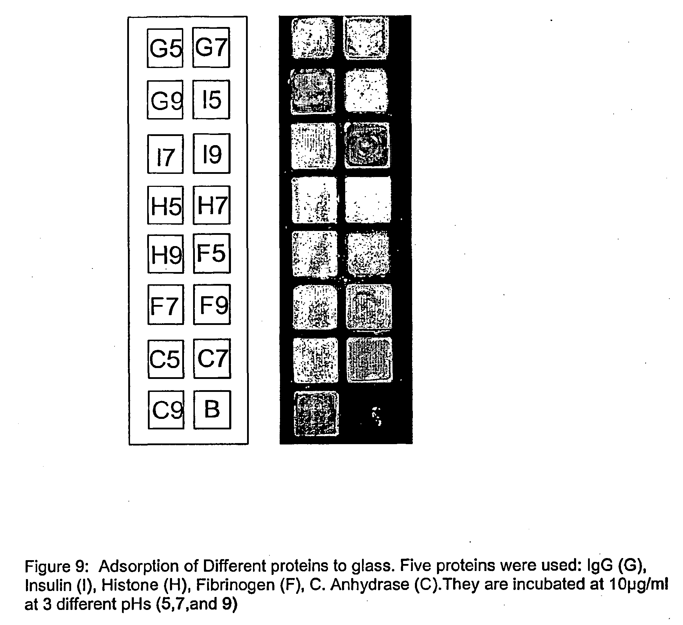

The results shown in figure 9 show that highly basic proteins (histone) and large proteins (fibrinogen) tend to adsorb the most. This makes sense considering the ionic attraction between the large amount of positive charges from histone, and the size of fibrinogen which allows for the simultaneous interaction of many residues of the protein at once.

### EXAMPLE V

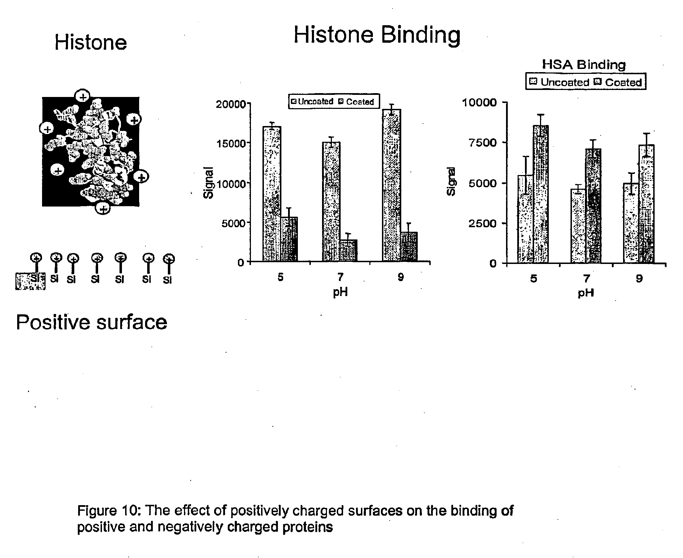

The effect of the surface charge will also modify the adsorption of the proteins. As the negative charges on glass tend to attract positively charged proteins. Positively charged surfaces should tend to repel them and attract negatively charged proteins.
Applying an aminosilane coating to the surface of the slides tests this theory. The coating results in a surface of packed amino groups that are protonated. The surface is then incubated with both basic (histone) and acidic (albumin) proteins. As can be seen in figure 10 the positively charged proteins adsorb less onto the positive surface, while the negatively charged proteins adsorb more when compared to a non-coated control.

### EXAMPLE VI

The optimization of the formulation of a protein therapeutic can consider many types of buffers at different pH and concentrations. The methods described within are aimed at increasing the throughput with which these variables are tested.

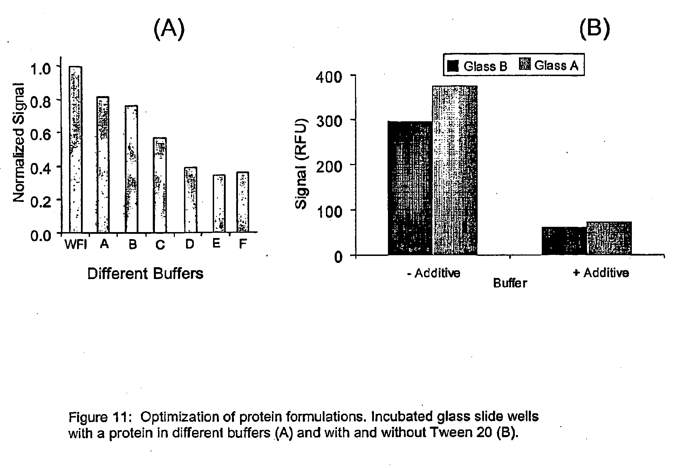

Protein solutions are made with different buffers and incubated in slide wells as describe in Example I. The proteins are then washed and the slides scanned. The results in Figure 11A demonstrate the effect of the different buffer compositions and concentrations when compared to incubating the wells with protein solutions in WFI (water for injection). It can be clearly seen that some buffers can reduce the adsorption of proteins to the surface by as much as 60%.

In another case the same protein solution is compared in terms of adsorption with and without the presence of a surfactant typically used in the pharmaceutical industry (Tween-20). The results in figure 11 B clearly show that the addition of the surfactant reduces the binding of the protein by at least 50%.

This example shows the utility of the methods in deterring protein adsorption, since just two multiplexed experiments can optimize the conditions to reduce the adsorption of the protein by a factor of 10.

In the foregoing and in the examples, all temperatures are set forth uncorrected in degrees Celsius and, all parts and percentages are by weight, unless otherwise indicated.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and can make various changes and modifications of the invention to adapt it to various usages and conditions.

## Claims

1. A multiplexed assay method capable of measuring the interaction of one or more biomolecule solutions with one or more substrate surfaces comprising:
contacting each of the wells of a multiwell substrate with the same or different biomolecule solutions, wherein the surfaces of said wells comprise
a plurality of different surface coatings and/or treatments to provide test surfaces, and
determining the level of biomolecule adsorption in each of said wells,
wherein said well surfaces comprise a plurality of different surface coatings and/or treatments.

2. A multiplexed assay according to claim 1, wherein said well biomolecule solution is selected from the group consisting of proteins, polypeptides, peptides, nucleic acids, polynucleotides and protein/nucleic acid complexes.

3. A multiplexed assay according to claim 2, wherein said protein, polypeptide or peptide is an antibody, an enzyme, a recombinant hormone, a recombinant interferon, a recombinant blood clotting cascade factor, a recombinant erythropoietin, a polypeptide or a vaccine antigen.

4. A multiplexed assay according to any one of claims 1 to 3, wherein said substrate is a glass slide or microtiter plate.

5. A multiplexed assay according to any one of claims 1 to 4, wherein the wells of said substrate are created with a hydrophobic patterning material applied to said substrate.

6. A multiplexed assay according to any one of claims 1 to 5, wherein said substrate contains from 2 to 10,000 individual wells.

7. A multiplexed assay according to claim 6, wherein said substrate contains from 10 to 1000 individual wells.

8. A multiplexed assay according to any one of claims 1 to 7, wherein said solutions contain a buffer, salt, stabilizer, preservative, acid and/or base.

9. A multiplexed assay according to claim 8, wherein said buffer is phosphate, citrate, and/or acetate.

10. A multiplexed assay according to claim 8, wherein said stabilizer is human albumin or glycerin.

11. A multiplexed assay according to claim 8, herein said preservative is phenol, metacresol or benzyl alcohol.

12. A multiplexed assay according to claim 8, wherein said acid or base is citric acid, sodium hydroxide, hydrochloric acid, or acetic acid.

13. A multiplexed assay according to any one of claims 1 to 12, wherein the level of biomolecule adsorption in each of said wells is determined by incubating the wells with a labeled antibody and scanning to determine the amount bound.

14. A multiplexed assay according to any one of claims 1 to 13, wherein the level of biomolecule adsorption in each of said wells is determined by interrogation with an enzyme conjugated antibody and measuring signal amplification.

15. A multiplexed assay according to any one of claims 1 to 14, wherein fluorescent detection is used as a direct indication of the amount of a biomolecule bound to a surface.

16. A multiplexed assay according to claim 15, wherein the biomolcule bound to said surface is in the form of a biomolecule:dye conjugate.

## Patentansprüche

1. Multiplex-Testverfahren zur Messung der Wechselwirkung von einer oder mehreren Biomolekül-Lösungen mit einer oder mehreren SubstratOberflächen, umfassend:
Inkontaktbringen jeder Vertiefung eines Substrats mit einer Mehrzahl an Vertiefungen mit derselben oder verschiedenen Biomolekül-Lösungen,
wobei die Oberflächen der Vertiefungen eine Mehrzahl von verschiedenen Oberflächenbeschichtungen und/oder -behandlungen umfassen, um Testoberflächen bereitzustellen,
und
Bestimmen des Grades der Adsorption der Biomoleküle in jeder Vertiefung, wobei die Oberflächen der Vertiefungen eine Mehrzahl von verschiedenen Oberflächenbeschichtungen und/oder -behandlungen umfassen.

2. Multiplex-Test nach Anspruch 1, wobei die Biomolekül-Lösung aus der Gruppe, bestehend aus Proteinen, Polypeptiden, Peptiden, Nukleinsäuren, Polynukleotiden und Protein/Nukleinsäure-Komplexen, ausgewählt ist.

3. Multiplex-Test nach Anspruch 2, wobei das Protein, Polypeptid oder Peptid ein Antikörper, ein Enzym, ein rekombinantes Hormon, ein rekombinantes Interferon, ein rekombinanter Blutgerinnungskaskade-Faktor, ein rekombinantes Erythropoietin, ein Polypeptid- oder ein Vakzin-Antigen ist.

4. Multiplex-Test nach einem der Ansprüche 1 bis 3, wobei das Substrat ein Glasträger oder eine Mikrotiter-Platte ist.

5. Multiplex-Test nach einem der Ansprüche 1 bis 4, wobei die Vertiefungen des Substrats durch ein Muster aus einem hydrophoben Material gebildet werden, das auf das Substrat aufgebracht wird.

6. Multiplex-Test nach einem der Ansprüche 1 bis 5, wobei das Substrat zwischen 2 und 10.000 einzelne Vertiefungen enthält.

7. Multiplex-Test nach Anspruch 6, wobei das Substrat zwischen 10 und 1.000 einzelne Vertiefungen enthält.

8. Multiplex-Test nach einem der Ansprüche 1 bis 7, wobei die Lösungen einen Puffer, ein Salz, einen Stabilisator, ein Konservierungsmittel, eine Säure und/oder eine Base enthalten.

9. Multiplex-Test nach Anspruch 8, wobei der Puffer Phosphat, Citrat und/oder Acetat ist.

10. Multiplex-Test nach Anspruch 8, wobei der Stabilisator menschliches Albumin oder Glycerin ist.

11. Multiplex-Test nach Anspruch 8, wobei das Konservierungsmittel Phenol, Meta-Kresol oder Benzylalkohol ist.

12. Multiplex-Test nach Anspruch 8, wobei die Säure oder Base Zitronensäure, Natriumhydroxid, Salzsäure oder Essigsäure ist.

13. Multiplex-Test nach einem der Ansprüche 1 bis 12, wobei der Grad der Adsorption der Biomoleküle in jeder Vertiefung durch Inkubation der Vertiefungen mit einem markierten Antikörper und Abtasten zur Bestimmung der gebundenen Menge bestimmt wird.

14. Multiplex-Test nach einem der Ansprüche 1 bis 13, wobei der Grad der Adsorption der Biomoleküle in jeder Vertiefung durch Abfrage mit einem Enzym-gekoppelten Antikörper und Messen der Signal-Verstärkung bestimmt wird.

15. Multiplex-Test nach einem der Ansprüche 1 bis 14, wobei eine Fluoreszenz-Detektion als direktes Maß für die Menge eines an eine Oberfläche gebundenen Biomoleküls verwendet wird.

16. Multiplex-Test nach Anspruch 15, wobei das an die Oberfläche gebundene Biomolekül in Form eines Biomolekül-Farbstoff-Konjugats vorliegt.

## Revendications

1. Procédé d'analyse multiplexe capable de mesurer l'interaction d'une ou de plusieurs solutions de biomolécules avec une ou plusieurs surfaces de substrat, consistant à :
mettre en contact chacun des puits d'un substrat multipuits avec des solutions de biomolécules identiques ou différentes, où les surfaces desdits puits comprennent une pluralité de revêtements et/ou de traitements de surface différents afin d'obtenir des surfaces de test,
et
déterminer le taux d'adsorption des biomolécules dans chacun desdits puits,
où lesdites surfaces des puits comprennent une pluralité de revêtements et/ou de traitements de surface différents.

2. Analyse multiplexe selon la revendication 1, dans laquelle ladite solution de biomolécules des puits est sélectionnée dans le groupe consistant en des protéines, des polypeptides, des peptides, des acides nucléiques, des polynucléotides et des complexes protéine/acide nucléique.

3. Analyse multiplexe selon la revendication 2, dans laquelle ledit/ladite protéine, polypeptide ou peptide est un anticorps, une enzyme, une hormone recombinante, un interféron recombinant, un facteur de la cascade de la coagulation sanguine recombinant, une érythropoïétine recombinante, un polypeptide ou un antigène vaccinal.

4. Analyse multiplexe selon l'une quelconque des revendications 1 à 3, dans laquelle ledit substrat est une lame en verre ou une plaque de microtitration.

5. Analyse multiplexe selon l'une quelconque des revendications 1 à 4, dans laquelle les puits dudit substrat sont créés avec un matériau à motifs hydrophobes qui est appliqué audit substrat.

6. Analyse multiplexe selon l'une quelconque des revendications 1 à 5, dans laquelle ledit substrat contient entre 2 et 10 000 puits individuels.

7. Analyse multiplexe selon la revendication 6, dans laquelle ledit substrat contient entre 10 et 1 000 puits individuels.

8. Analyse multiplexe selon l'une quelconque des revendications 1 à 7, dans laquelle lesdites solutions contiennent un tampon, un sel, un stabilisateur, un conservateur, un acide et/ou une base.

9. Analyse multiplexe selon la revendication 8, dans laquelle ledit tampon est le phosphate, le citrate et/ou l'acétate.

10. Analyse multiplexe selon la revendication 8, dans laquelle ledit stabilisateur est l'albumine humaine ou la glycérine.

11. Analyse multiplexe selon la revendication 8, dans laquelle ledit conservateur est le phénol, le métacrésol ou l'alcool benzylique.

12. Analyse multiplexe selon la revendication 8, dans laquelle ledit acide ou ladite base est l'acide citrique, l'hydroxyde de sodium, l'acide chlorhydrique, ou l'acide acétique.

13. Analyse multiplexe selon l'une quelconque des revendications 1 à 12, dans laquelle le taux d'adsorption des biomolécules dans chacun desdits puits est déterminé par une incubation des puits avec un anticorps marqué et par un balayage afin de déterminer la quantité liée.

14. Analyse multiplexe selon l'une quelconque des revendications 1 à 13, dans laquelle le taux d'adsorption des biomolécules dans chacun desdits puits est déterminé par une interrogation avec un anticorps conjugué à une enzyme et par une mesure de l'amplification du signal.

15. Analyse multiplexe selon l'une quelconque des revendications 1 à 14, dans laquelle une détection fluorescente est utilisée en tant qu'indication directe de la quantité de biomolécule liée à une surface.

16. Analyse multiplexe selon la revendication 15, dans laquelle la biomolécule liée à ladite surface est sous la forme d'un conjugué biomolécule:colorant.
